(19) Europäisches Patentamt
European Patent Office
Office européen des brevets

(11) Publication number: **0 566 066 A1**

(12) **EUROPEAN PATENT APPLICATION**

(21) Application number: **93106003.2**

(22) Date of filing: **30.10.85**

(51) Int. Cl.[5]: **C07K 15/06**, A61K 39/00, //C12P21/08,G01N33/577

This application was filed on 13 - 04 - 1993 as a divisional application to the application mentioned under INID code 60.

(30) Priority: **02.11.84 US 667521**
**07.10.85 US 785177**

(43) Date of publication of application:
**20.10.93 Bulletin 93/42**

(60) Publication number of the earlier application in accordance with Art.76 EPC: **0 207 090**

(84) Designated Contracting States:
**AT BE CH DE FR IT LI LU NL SE**

(71) Applicant: **ONCOGEN LIMITED PARTNERSHIP**
**3005 First Avenue**
**Seattle, WA 98121(US)**

(72) Inventor: **Hellstrom, Ingegerd**
**3925 North East Surber Drive**
**Seatlle, WA 98121(US)**
Inventor: **Brown, Joseph Patrick**
**409 Newton Street**
**Seatlle, WA 98121(US)**
Inventor: **Hellstrom, Karl Erik**
**3925 North East Surber Drive**
**Seatlle, WA 98121(US)**
Inventor: **Horn, Diane**
**202 West Olympic Place, 404**
**Seatlle, WA 98121(US)**
Inventor: **Linsley, Peter**
**2430 Ninth West**
**Seatlle, WA 98121(US)**

(74) Representative: **Warcoin, Jacques et al**
**Cabinet Régimbeau**
**26, avenue Kléber**
**F-75116 Paris (FR)**

(54) **Antigens of human non-small lung carcinoma.**

(57) An antigenic composition purified from normal human serum or the culture medium of human non-small cell lung carcinoma cells comprising a non-denatured glycosylated Form I L3 protein, said protein having the following N-terminal amino acid sequence :

```
1       5         10        15        20        25        30
V-N-D-G-D-M-R-L-A-D-G-G-A-T-N-Q-G-R-V-E-I-F-Y-R-G-Q-W-G-T-V,
```

a molecular weight of about 94,000 daltons as determined by SDS polyacrylamide gel electrophoresis under reducing conditions, and N-linked glycoside residues in which the terminal residues of the N-linked glycoside residues are sialic acid residues.

EP 0 566 066 A1

## BACKGROUND OF THE INVENTION

### 1. Field of the Invention

Human lung carcinomas are responsible for most deaths from cancer among men and are in the process of overtaking breast carcinomas as the most frequent cause of cancer death among women (Cancer Facts and Figures, 1983). This disease can be divided into 4 major histological types, i.e., epidermoid (30%), adenocarcinoma (35%), large-cell undifferentiated (15%), and small-cell (20%). Most cases of lung carcinomas are incurable by chemotherapy and radiation therapy. Small cell lung carcinomas may respond to chemotherapy and radiation therapy by a reduction in size, but not a total cure. Complete surgical removal of the tumor appears to be the only effective therapy. Unfortunately, however, fewer than 30% of lung cancer patients have tumors which can be totally resected at diagnosis and of these, fewer than one-third survive 5 years after apparent complete surgical removal of all tumor. There is a great need, therefore, for methods that would make possible an earlier diagnosis of lung cancer, a better definition of the degree of cancer spread, and a more effective therapy.

Monoclonal antibodies may be used for all these purposes. A prerequisite, however, is to find antibodies to antigens that are more strongly expressed in lung cancer than in normal adult tissues. In view of the known heterogeneity of tumor cell populations, the presence of several determinants on the some antigen molecule, the anticipated differences between antigens with respect to their suitability as diagnostic markers as compared to therapeutic targets, and the different biological characteristics of different antibodies to the same antigen, a number of different antibodies may be needed.

### 2. Description of the Prior Art

Monoclonal antibodies to lung cancer antigens are described by Sikora et al., Br. J. Cancer (1981) 43:696-700; Cuttitta et al., Proc. Natl. Acad. Sci. U.S.A. (1981) 78:4591-4595; Varki et al., Cancer Research -(1984) 44:681-687; Moody, T.W. et al., Science, (1981) 214:1246-1248; Minna, J.D. et al., In Vitro 17(12)-:1058-1070 (12-1981); Kennel, A.J. et al., Cancer Research, 41(9,PT1):3465-3470. Chem. Abst. 95(15)-:1308502; Baylin, A.B. et al., Proc. Natl. Acad. Sci. U.S.A. 79:4650-4654 (8-1982); Carney, D.N. et al., Pathobiology Annual 1982, 12:115-136 (1982); Gazdar, A.F. et al., Seminars in Oncology, 10:3-19(3-1983); Hollinshead, A.C. et al., Cancer Detec. Prevent., 6:185-191 (1983); Mulshine, J.T. et al., J. Immunol., 131(1)-:497-502 (1983); Huang, L.C. et al., Arch. Bioch. Biophys., 220(1):318-320 (1983); Saji, S. et al., Hybridoma, 3(2):119-130 (1984), Bio. Abst. 79005569; Bosslet, K. et al., Behring Inst. Mitt., 74:27-34 (1984), Chem. Abst. CA101(9):70668b; Rosen, A.T. et al., Cancer Research, 44(5):2052-2061 (1984), Bio. Abst. 79014658; Van Muijen, G.N.P. et al., Amer. J. Pathol., 116(3):363-369 (1984), Bio. Abst. 79023605; Princler, G.J. et al., Cancer Research, 42:843-848 (3-1982); Mazauric, T. et al., Cancer Research, 42:150-154 (1-1982); Braatz, J.A. et al., Cancer Research, 42:849-855 (3-1982); and Sobol, R.E. et al, Fed. Proc., 41(3):409. Abst. 816 (4-1982).

Continuous cultures of fused cells secreting antibody of predefined specificity are described by Köhler et al., Nature (1975) 265:495-497. Methods of producing tumor antibodies are described in U.S. Patent No. 4,172,124. T. Mazauric et al., Cancer Research Vol. 42 (1982), pages 150-154, discloses monoclonal antibodies which define four distinct antigens on the surface of human lung tumors. P. Abrams et al., Proc. Am. Assoc. Cancer Research Clin. Oncol., Vol. 22 (1981), page 373, n° C-163 discloses the fusion of mouse myeloma cells and B. lymphocytes from BALB/c mice immunized with an established line of human small cell lung carcinoma cells CNCI-H125.

## SUMMARY OF THE INVENTION

The present invention is concerned with novel antigen compositions and monoclonal antibodies which define determinant sites on such antigens of human non-small cell lung carcinoma (NSCLC) cells as defined in claims 1-10. The term "NSCLC cells" includes epidermoid carcinoma cells, adenocarcinoma cells, and large cell undifferentiated carcinoma cells. The determinant sites may also be found on antigens of some other carcinomas, e.g. some carcinomas of the breast, and, thus, the antibodies of the invention will also bind to these other carcinoma cells. These monoclonal antibodies bind to a lesser degree to normal adult cells than to tumor cells. The term "bind to a lesser degree" means that the binding will not be detectable by immunohistological techniques or that the binding will be about four to five times less than the binding to NSCLC cells as determined by immunohistological techniques. The monoclonal antibodies are secreted by murine hybridomas.

The invention also concerns certain diagnostic methods employing the monoclonal antibodies or antigen compositions of the invention. One such method involves the determinant of the presence of NSCLC cells in a specimen suspected of containing such cells. The specimen is contacted with the monoclonal antibody, which is capable of distinguishing such cells from other cell types which may be present in the specimen. The contact is carried out under conditions for binding of the antibody to such cells. After contact, the presence or absence of binding of the antibody to the cells in the specimen is determined. This binding is related to the presence or absence of the NSCLC cells in the specimen. Generally, the specimen is contacted with a labeled specific binding partner for the monoclonal antibody. This label is capable of producing a detectable signal.

More particularly, the present invention provides antigenic compositions such as claimed in claims 1 to 3.

## DESCRIPTION OF THE SPECIFIC EMBODIMENTS

The present invention concerns certain novel antigens in purified form and antibodies specific for such antigens on human NSCLC cells, and functional equivalents, binding fragments and immunocomplexes of such antibodies and certain diagnostic methods employing such antibodies. For example, the monoclonal antibodies of the invention may be produced according to the standard techniques of Köhler and Milstein, supra. For example, human lung carcinoma cells from plural effusions or cultured cells from human non-small cell lung carcinoma, or cells from a normal fetal lung, are used as the immunogen. These cells are injected into a mouse and, after a sufficient time, the mouse is sacrified and spleen cells obtained. The spleen cell chromosomes encoding desired immunoglobulins are immortalized by fusing the spleen cells with myeloma cells or with lymphoma cells, generally in the presence of polyethylene glycol. The resulting cells, which include the fused hybridomas, are allowed to grow in a selective medium, such as HAT-medium, and the surviving cells are grown in such medium using limiting dilution conditions. The cells are grown in a suitable container, e.g., microtiter wells, and the supernatant is screened for monoclonal antibodies having the desired specificity.

Various techniques exist for enhancing yields of monoclonal antibodies, such as injection of the hybridoma cells into the peritoneal cavity of a mammalian host, which accepts the cells, and harvesting the ascites fluid. Where an insufficient amount of the monoclonal antibody collects in the ascites fluid, the antibody is harvested from the blood of the host. Various conventional ways exist for isolation and purification of the monoclonal antibodies, so as to free the monoclonal antibodies from other proteins and other contaminants (see Köhler and Milstein, supra).

One such monoclonal antibody of the present invention is exemplified by a novel antibody designated L3. This monoclonal antibody defines a determinant site on a cell associated antigen of Mr 76,000 by SDS-PAGE characteristic of human NSCLC cells, i.e. malignant cells, and a determinant site on protein antigens that are secreted into the culture medium by the NSCLC cells (Form I, II and III antigen). The antibody is of the $IgG_1$ isotype. The L3 antibody binds to a lesser dregree to some normal cells. The L3 antibody is produced by the L3 murine hybridoma.

Form I L3 antigen has an Mr of 94,000 by sodium dodecyl sulfate-polyacryamide one-dimensional gel electrophoresis (SDS-PAGE). Form II L3 antigen has an Mr of 76,000 by SDS-PAGE and Form III L3 antigen has an Mr of 26,000 by SDS-PAGE.

Form I L3 antigen can be purified ninety fold from culture medium by affinity chromatography (Table I) and affinity chromatography followed by acrylamide gel techniques to give compositions containing greater than 70% and 90% (by weight), respectively, of the Form I L3 antigen which is a glycoprotein. The composition obtained from affinity chromatography purification has a Specific Activity of 46,500 Units/mg as compared to 522 Units/mg for the culture medium. The Specific Activity is defined as the amount of antigen required to inhibit binding of $^{125}$I-labeled L3 antibody by 50% in a competitive binding assay employing formalin fixed monolayers of Calu-1 cells. The composition obtained from the culture medium by affinity chromatography contains greater than 70% Form I antigen and for the most part less than 30% of unspecified protein material of Mr between about 50,000-200,000 by SDS-PAGE including minor amounts, about 10 to 20% of Form II antigen. Also present in the composition are trace amounts of Form III antigen.

In order to determine the relationship between the cellular and extracellular forms of the L3 antigen, cells were radiolabeled with $^{35}$S methionine and a pulse chase experiment was performed. Cells were pulse labeled for one hour with $^{35}$S-methionine, placed into medium containing unlabeled methionine for various times and subjected to immunoprecipitation analysis. Initially, all specificially immunoprecipitable cell-associated radioactivity was found in an Mr 76,000 protein. The other labeled proteins seen were also found in samples from which antibody was omitted. During the chase period, radioactivity in the Mr 76,000 form decreased while radioactivity found in the form released into the medium (Mr 94,000) increased. Radioactivity in the cell-associated form disappeared with the same kinetics as radioactivity in the released form appeared; the $t_{1/2}$ for both processes was determined by densitometric analysis to be approximately 1.5 hrs. These results suggest that the cell associated Mr 76,000 form of the antigen is a precursor of the Mr 94,000 form (Form I L3 antigen).

Since the L3 antigen is a glycoprotein, carbohydrate modifications might conceivably account for the apparent increase in size of the L3 antigen observed during its release from the cell. This possibility was examined by treating immunoprecipitates containing the cellular and released forms of the molecule with glycosidases of known specificity. The size of the cell associated L3 antigen was reduced to Mr 59,000 by treatment with endoglycosidase H (Endo H) but was unaffected by neuraminidase, indicating that it contained N-linked high mannose oligosaccharides (see Tarentino et al, 1974, J. Biol. Chem., 249:811-817). This result is consistent with the finding that maturation is sensitive to tunicamycin, a know inhibitor of N-linked glycosylation (Lehle, et al., 1976, FEBS Letters, 71:167-170). In contrast, the released antigen was Endo H resistant, but was reduced in size to Mr 80,000 by neuraminidase treatment, indicating the presence of terminal sialic acid residues on Form I L3 antigen. Thus, the conversion of L3 antigen from a cell-associated to a released form is accompanied by maturation of N-linked carbohydrate side chains.

The amino acid terminal sequence of the Form I L3 antigen was determined by conventional techniques on the composition purified by a combination of affinity chromatography and acrylamide gel separation techniques. This sequence (α) is as follows:

```
1        5         10        15
V-N-D-G-D-M-R-L-A-D-G-G-A-T-N-Q
```

and is further defined as follows:

```
       20        25        30
α-G-R-V-E-I-F-Y-R-G-Q-W-G-T-V
```

The above one letter symbols for the amino acids have their conventional meaning where

V = valine, N - asparagine, D - aspartic acid, G = glycine, M = methionine, R = arginine, L = leucine, A = alanine, T = threonine, Q = glutamine, E = gluatmic acid, I = isoleucine, F = phenylalanine, Y = tyrosine, and W = tryptophan. The above is obtained as a single sequence for the composition containing Form I L3 antigen whether or not Form II L3 antigen is present.

Comparison of the above 30 N-terminal amino acids of the L3 antigen with protein sequences present in the National Biomedical Research Foundation protein bank (March 1985 edition) revealed no significant homologies. The sequence of the Form I L3 antigen was also compared with that of several serum proteins not currently listed in this data base, including human Cl-esterase inhibitor, and human alpha-2-thiolproteinase inhibitor. None of these sequences revealed any significant homologies with the N-terminal sequence of the Form I L3 antigen.

We determined that the Form I L3 antigen is present in normal human serum and purified the Form I L3 antigen from serum. The properties of the L3 antigen from serum are summarized in Table III. The Form I L3 antigen can be purified more than 3,000-fold from normal human serum with a yield of 39% (Table II). Analysis of the purified material by SDS PAGE reveals the major components to be proteins which co-migrated with the Form I and Form II components purified from culture medium; neither form is a major component of the initial serum sample. It is noteworthy that both Form I and II L3 antigens from serum are resolved into two distinct components on the original gel, possibly indicating microheterogeneity due to glycosylation differences. Both forms are reactive following immunoblotting analysis and after radioiodination and immunoprecipitation. As with the antigen purified from culture medium, an additional form Mr 26,000 (Form III L3 antigen) is detected after immunoprecipitation. The composition obtained by immunoaffinity chromatography contains greater than 50% by weight of Form I L3 antigen, the remaining material being Form II L3 antigen, transferrin and unspecified proteins of Mr about 50,000-200,000 by SDS-PAGE. This composition has a Specific Activity of 45,500 Units/mg as compared to 13.3 Units/mg for normal human serum.

These results indicate that L3 antigenic activity is expressed on two components (Form I and II) found in both culture medium and serum. Further purification of the Form I L3 antigen from serum by preparative SDS-PAGE resulted in compositions which contained predominantly Form I L3 antigen at a level greater than 90% by weight and variable trace amounts of Form II antigen with the remaining material being transferrin and unspecified proteins of Mr about 50,000 - 200,000.

$^{125}$I-labeled and immunoprecipitated antigens purified from both serum and culture medium by two dimensional isoelectric focusing - SDS PAGE were compared. The two dimensional mobilities of both antigens are closely similar, with both Form I and Form II components migrating as heterogeneous species in the pH range of 4.2-5.0. Form I L3 antigen from both sources also reveal two minor components migrating at approximate pH values or 5.8 and 6.2. The carbohydrate composition of both antigens was also examined by testing for glycosidase sensitivity. Form I and Form II L3 antigens from both sources are found to be Endo H resistant, while neuraminidase treatment of both resulted in the appearance of the characteristic Mr 80,000 fragment seen following treatment of metabolically labeled antigen. The neuraminidase sensitivity of Form II antigen indicates that it is different from the Mr 76,000 cell associated form of the L3 antigen.

To confirm that both molecules were structurally similar, we subjected Form I molecules from both sources to a series of partial protease cleavage reactions as described by Cleveland et al., (1977) J. Biol. Chem., 252:1102-1106. The radiolabeled Form I antigens purified from both culture medium and serum are excised from a preparative SDS-PAGE gel and subjected to fingerprinting with V8 protease. Both molecules yield identical partial cleavage patterns; at least five dinstinctive partial cleavage products are observed (Mr = 76,000, 49,000, 31,000, 20,000 and 14,300) in both cases. These results demonstrate that L3 antigens purified from culture medium and human serum are highly related.

We next investigated the relationship between the three molecular weight forms of L3 antigen from serum. The overall fingerprints for Forms I and II are quite similar, although the Form II molecule did not yield the Mr 31,000 and Mr 14,300 fragments derived from the Form I molecule. The patterns suggest that Form I and Form II molecules arc closely related. The Form III antigen shares a Mr 14,300 partial cleavage product with the larger antigenic forms found in serum, which may indicate that the Form III antigen is also structurally related to Form I antigen. The close similarities in structure between the various forms suggest that Forms II and III molecules are derived from Form I antigen.

To determine whether the L3 antigen was described previously as an antigenic component of a tumor type other than carcinoma of the lung, a computer assisted literature search for shed or secreted tumor associated antigens was performed. We discovered several reports of melanoma-secreted protein antigens which were of similar size and carbohydrate composition as the L3 antigen. Natali et al., (1982) Cancer Res. 42:583-589; Bumol et al., (1982) Hybridoma 1:283-292; and Wilson et al., (1981) Int. J. Cancer 28:293-300.

Small amounts of one of the previously described monoclonal antibodies (465.12S; Natali et al, supra) was available to us through our participation in the First International Workshop on Melanoma Associated Antigens. We employed this antibody in a sequential immunoprecipitation experiment to test for identity between the L3 antigen and the antigen recognized by antibody 465.12S. Both antibodies specifically precipitated a Mr 94,000 protein species from culture medium of cells metabolically labeled with $^{35}$S-methionine. Depletion of the culture medium for either L3 or 465.12S antigens by immunoprecipitation resulted in a concomitant depletion of antigenic activity of the alternative antigen. This shows that the antigenic determinants recognized by both antibodies are carried on the same molecule (Table III). However, the epitopes recognized by the two antibodies are apparently different since we were unable to detect competition by antibody 465.12S for binding or $^{125}$I-L3 antibody.

Another monoclonal antibody of the invention, designated L5, defines a determinant site on a cell surface protein antigen characteristic of human NSCLC cells. The molecular weight of the protein, as determined by the method described above and by a method based on $^{125}$I labelling is about 135,000 daltons. This antibody is of the IgM class. The L5 antibody does not bind detectably to normal cells, such as fibroblasts, endothelial cells, and epithelial cells in the major organs. The L5 antibody is produced by the L5 murine hybridoma. The L5 antigen has two characteristic V8 protease fragments of Mr 24,000 and 16,000 as shown by a technique described by Cleveland et al. (1977) J. Biol. Chem. 252:1102-1106. Thus, the L5 antigen is distinguished from other antigens or similar molecular weight.

Also included within the scope of the invention are useful binding fragments or the monoclonal antibodies above such as Fab, $F(ab')_2$, Fv fragments and so forth. The antibody fragments are obtained by conventional techniques. For example, useful binding fragments may be prepared by peptidase digestion of the antibody using papain or pepsin. By the term useful binding, fragment is meant that the fragment will compete with an antibody for binding sites on its cognate antigen.

While the above specific examples of the novel antibodies of the invention are directed to antibodies binding to specific determinant sites on the respective antigens and being of specific classes and isotypes from a murine source, this is not meant to be a limitation. The above antibodies and those antibodies having functional equivalency with the above antibodies, whether from a murine source, mammalian source including human, or other sources, or combinations thereof are included within the scope of this invention, as well as other classes such as IgM, IgA, IgE, and the like, including isotypes within such classes. By the term "functional equivalency" is meant that the antibody is capable of binding to the above-described determinant site and capable of competing with a particular antibody of the invention for such site. That is, such antibody, when combined with a specimen containing a cell or cell fragment having such determinant site, will bind to such determinant site and will block an antibody of the invention from binding to such site.

One method of the invention involves the determination of the presence of a malignant condition in lung tissue. The term "malignant condition" refers to the presence of dysplastic including carcinoma in situ, neoplastic, malignant, or tumor cells, or the like. The specimen is contacted or combined with a monoclonal antibody of the invention. The contact is carried out under conditions for binding of the antibody to the malignant cells. After contact, the presence of binding of the antibody to the malignant cells in the specimen is observed. That is, the specimen is examined for immune complexes of the antibody and the antigenic site. This immune complex formation is related to the presence of malignant cells in the specimen.

A particular example, by way of illustration and not limitation, of a method in accordance with the invention is a method for the detection of tumor cells in excised tissue. The above method is applied to a specimen which is a section of the tumor obtained after removal of the tumor. The tumor that is excised is treated to obtain sections, which treatment initially involves freezing the tumor or tissue, normally freezing immediately after excision. The frozen layer of tissue is then cut into sections using, for example, a cryostat.

The section of the tumor obtained as described above is contacted with a monoclonal antibody of the invention and then with a second antibody directed against the above monoclonal antibody, which second antibody is labeled with a detectable label.

The excised specimen, e.g., the section of the tumor, is contacted with the first monoclonal antibody under conditions for binding of the antibody to the malignant cells. The incubation is generally conducted in an aqueous medium such as, for example, phosphate buffered saline containing a small amount of sodium azide, in a suitable container such as, for example, a glass petri dish, for a period from about 15 to 30 minutes at a temperature of from about 20 to 30 °C. The amount of antibody employed is usually sufficient to provide detectable binding, i.e., to provide a detectable number of immune complexes between the antibody and the determinant or antigenic site in question.

Following the incubation, the section is washed to reduce or eliminate non-specifically bound antibody and then is examined to observe the above-mentioned complexes which result from binding of the monoclonal antibody to the cells of the specimen possessing the antigenic site. The binding is related to the presence of malignant cells in the section. Accordingly, binding is determined, for example, by contacting the specimen with a labeled specific binding partner for the monoclonal antibody. The label is capable of producing a detectable signal and may be a radioactive label, a chromophore such as a fluorescer or an enzyme.

An example of a technique employing the above approach is immunofluorescence staining. In this technique frozen sections of the tumor are fixed on a glass slide with acetone and are incubated with the monoclonal antibody in, for example, a petri dish. After washing with an appropriate buffer such as, for example, phosphate-buffered saline, the section is placed on a petri dish and contacted with the labeled specific binding partner for the monoclonal antibody, which may be, for example, a labeled antibody specific for the monoclonal antibody employed. Since, for the most part, the monoclonal antibody will be derived from a murine source, a labeled antimouse immunoglobulin specific for the monoclonal antibody may be employed. Such immunoglobulins may be raised according to standard techniques by injecting a suitable host with murine antibody, waiting for an appropriate time, and harvesting the antimouse immunoglobulins from the blood of the injected host.

After a second washing of the slide with, for example, an aqueous buffer, the sections may be covered with a fluorescent antibody mounting fluid and a coverslip and then examined with a fluorescence microscope to determine the binding of the monoclonal antibody to the section. The determination of the binding also may include an identification of the location of such binding within the specimen.

The binding of the monoclonal antibody to the specimen may also be determined by employing a monoclonal antibody which is covalently conjugated to a label capable of producing a detectable signal, such as a radioactive entity, a chromophore including dyes and fluorescers, or an enzyme. The number of labels employed per antibody is generally determined by the requirements of the diagnostic method in which the labeled antibody is employed and the availability of sites for linking the label to the antibody.

Methods for conjugating labels to antibodies and antibody fragments are well-known in the art. Such methods may be found in U.S. Patent Nos. 4,220,450; 4,235,869; 3,935,074; and 3,996,345.

Another example of a technique in which the monoclonal antibody of the invention may be employed is immunoperoxidase labeling (Sternberger, Immunocytochemistry, John Wiley & Sons, New York, 1979, pp:104-169 as modified by Garrigues et al., Int. J. Cancer (1982) 29:511-515). The tissue to be tested is fixed with a suitable solvent, such as acetone, on a support, such as a glass slide. Next, the tissue is incubated with the monoclonal antibody and then washed free of unbound antibody. Then, the tissue is incubated with rabbit anti-mouse IgG, washed to remove unbound antibody, combined with mouse peroxidase-anti-peroxidase complex, washed to remove unbound conjugate, and then treated with substrate for the enzyme. Following this treatment the slide is examined for a detectable signal.

The antibodies of the invention may be used in a method of determining the presence of a malignant condition in an exfoliative cell specimen from the lung, such as sputum. By the term "exfoliative" is meant that the specimen comprises isolated cells or clumps of cells obtained by scraping or washing the surface of tissue, which cells are removed individually or in scales or laminae. The exfoliative cell specimen is to be distinguished from excised tissue such as that obtained by biopsy. Contact between the specimen and the antibody is made under conditions for binding of the antibody to the antigenic site. After contact, the presence or absence of binding of the antibody to the antigenic site is determined and is related to the presence of a malignant condition in the lung.

To determine the presence of a malignancy in the lung, a sputum sample would provide the exfoliative cell specimen to be used in the method. The method may find utility in the detection of a malignant condition in exfoliative cell specimens from the bronchus, gastro-intestinal tract including oral pharynx, mouth, etc.

The exfoliative cell specimen is next contacted with the aforementioned antibody under conditions for binding of the antibody to the specific antigenic site in the specimen to form antigen-antibody complexes. This antigenic site may be present on cells or cell fragments in the specimen. Generally, the specimen is placed on an appropriate support, such as, for example, a slide, usually glass, or some other suitable material. The exfoliative cell specimen is generally smeared on the slide to provide a thin layer of the specimen on the surface of the slide. The contact between the antibody and the specimen is generally carried out in an aqueous buffered medium. The buffers which may be employed include phosphate, tris, bicarbonate, etc. The pH is related to the nature of the specimen and the antibody, and is generally in the range of from about 5 to 8. The aqueous medium may additionally contain organic polar solvents in an amount of from about 0 to 40%. The organic polar solvents are water soluble and generally have from about 1 to 10 carbon atoms and from about 1 to 4 oxygen atoms. The antibody will be present in the aqueous medium at a concentration of about 1 to 100 µg/ml, preferably from about 10 to 20 µg/ml. The temperature during the contact of the specimen with the antibody is usually from about 4 to 40°C, preferably about 10 to 30°C. The period of contact is usually from about 15 to 120 minutes, preferably from about 30 to 60 minutes.

After the period of contact between the specimen and the antibody, the support is generally treated to remove unreacted antibody. Normally, this is accomplished by washing the support with an aqueous, usually buffered, medium. In general, the amount of wash solution should be sufficient to remove the unreacted antibody.

Next, the presence of binding of the antibody to the antigenic site in the specimen, which binding is related to the presence of a malignant condition at the locus, is observed. That is, the specimen is examined to determine the number of antigen-antibody (immune) complexes formed. It should be noted that in some instances very small numbers of the antigenic site in question may be found in the exfoliative cell specimen. However, in a malignant condition, large numbers of the antigenic site will be present and this latter condition is readily distinguishable by this method over a non-malignant condition because a large number of antigen-antibody complexes will be measurable where a malignant condition exists. To make the determination of the presence of binding, means for producing a detectable signal is incorporated into the assay system. For example, one may conjugate the antibody employed in the assay to a label which is capable of producing a detectable signal. The label may be a radioactive entity, a chromophore including dyes and fluorescers or an enzyme. The number of labels employed for the antibody is generally determined by the requirements of the method and the availability of sites for linking the label to the antibody.

Alternatively, one may contact the washed slide with a labeled specific binding partner for the antibody, which may be, for example, a labeled antibody specific for the antibody employed. Where the monoclonal antibody is derived from a murine source, a labeled anti-mouse immunoglobulin specific for the antibody employed in the method may be used. Such immunoglobulins may be raised according to standard techniques by injecting a suitable host with the monoclonal antibody, waiting for an appropriate time, and harvesting the anti-mouse immunoglobulins from the blood of the injected host. When a labeled specific binding partner for the antibody is employed, the slide must be washed again with an aqueous medium prior to examining the slide for fluorescence.

To determine the presence of binding between the antibody and the cell specimen where a fluorescer label is used, one may examine the slide for fluorescence, usually employing a fluorescence microscope. Where a label other than a fluorescer is employed, one may examine the slide or the specimen for the formation of a precipitate or a color, for instance.

The above description is directed primarily to the use of the antibodies of the invention in immunofluorescence techniques. However, the antibodies of the invention may be used in most assays involving antigen-antibody reactions. The assays may be homogeneous or heterogeneous. In a homogeneous assay approach, the specimen may be biological fluid such as serum, urine, and the like or the specimen may be lysed and clarified to remove debris. Antibody L3, for example, has been used by us to measure the concentration of its cognate antigen, i.e., Form I, II, or III L3 antigen, in serum from cancer patients, and certain cancer patients have been found to have elevated levels of this antigen when compared to normal controls. The immunological reaction usually involves the specific antibody, a labeled analyte, and the sample of interest. The signal arising from the label is modified, directly or indirectly, upon the binding of the antibody to the labeled analyte. Both the immunological reaction and detection of the extent thereof can be carried out in a homogeneous solution. Immunochemical labels which may be employed include free radicals, fluorescent dyes, enzymes, bacteriophages, coenzymes, and so forth.

In a heterogeneous assay approach, the reagents are usually the specimen, the specific antibody, and means for producing a detectable signal. The specimen is generally placed on a support, such as a plate or a slide, and contacted with the antibody in a liquid phase. The support is then separated from the liquid phase and either the support phase or the liquid phase is examined for a detectable signal employing means for producing such signal. The signal is related to the presence of the analyte in the specimen. Means for producing a detectable signal includes the use of radioactive labels, fluorescers, enzymes, and so forth. Exemplary of heterogeneous immunoassays are the radioimmunoassay, immunofluorescence methods, and enzyme-linked immunoassays.

For a more detailed discussion of the above immunoassay techniques, see "Enzyme-Immunoassay," by Edward T. Maggio, CRC Press, Inc., Boca Raton, Florida, 1980. See also, for example, U.S. Patent Nos. 3,690,834; 3,791,932; 3,817,837; 3,850,578; 3,853,987; 3,867,517; 3,901,654; 3,935,074; 3,984,533; 3,996,345; and 4,098,876, which listing is not intended to be exhaustive.

The antibodies of the invention can also be employed to image metastic deposits in human patients with NSCLC in a manner analogous to that described for malignant melanoma in J. Nucl. Med. (1983) 24:123-129 and in J. Clin. Invest. (1983) 72:2101-2114. The antibody or fragments thereof are radiolabelled and adminstered intraveneously to a patient who subsequently is imaged using, e.g., a gamma camera or the like.

The invention also includes diagnostic kits for carrying out the methods disclosed above. In one embodiment, the diagnostic kit comprises in a packaged combination (a) a monoclonal antibody more specifically defined above and (b) a conjugate of a specific binding partner for the above monoclonal antibody and a label capable of producing a detectable signal. The reagents may also include ancillary agents such as buffering agents and protein stabilizing agents, e.g., polysaccharides and the like, as required to conduct such methods. The diagnostic kit may further include, where necessary, other members of the signal producing system of which system the label is a member, agents for reducing background interference in a test, control reagents, apparatus for conducting a test, and the like. In another embodiment, the diagnostic kit comprises a conjugate of a monoclonal antibody of the invention and a label capable of producing a detectable signal. Ancillary agents as mentioned above may also be present.

The antibodies of the invention may be used therapeutically. Antibodies with the proper biological properties are useful directly as therapeutic agents. Alternatively, the antibodies can be bound to a toxin to form an immunotoxin or to a radioactive material or drug to form a radiopharmaceutical or pharmaceutical. Methods for producing immunotoxins and radiopharmaceuticals of antibodies are well-known (see, for example, Cancer Treatment Reports (1984) 68:317-328).

Another therapeutic use of the monoclonal antibodies of the present invention is the immunization of a patient with an anti-idiotypic antibody raised by using one of the present monoclonal antibodies as an immunogen. Such immunization can induce an active anti-tumor activity (see, for example, Nepom et al.; Proc. Natl. Acad. Sci. U.S.A. (1984) 81:2864-2867. In a similar approach, the patient can be immunized with the antigen in purified form, a peptide fragment of the antigen, or a modified form of the antigen.

A particularly attractive aspect of the present invention is that the L3 antibody can be used in combination with other antibodies. The combination can be effective in detecting at least the types of lung carcinomas mentioned above, namely, large cell undifferentiated lung carcinoma, adenocarcinoma, and epidermoid carcinoma. For example, monoclonal antibodies L3 (disclosed in U.S. Serial No. 667,521, filed November 2, 1984) can be combined in effective amounts, i.e., amounts that will render the combination capable of detecting all of the aforementioned lung carcinomas.

Some of the monoclonal antibodies of the invention also define determinant sites on antigens associated with other carcinomas such as breast carcinoma and epidermoid carcinomas of the vulva. Consequently, the present antibodies can find use in diagnostic and therapeutic products directed to such carcinomas.

## EXAMPLES

The invention is further demonstrated by the following illustrative Examples. A number of procedures employed will be described first.

### Materials

Culture media were from Gibco. Fetal bovine serum was from Gibco or Hyclone. Teflon coated multiwell microscope slides (12 well) were from Meloy. Plastic culture dishes were from Corning or Costar. V8 protease and bovine serum albumin were from Sigma. Neuraminidase and Pansorbin (formalin-fixed S. aureus) were from Calbiochem. Endoglycosidase H was from Miles. Nitrocellulose (BA85, 0.45u) was from Schleicher and Schuell. Protein A - Sepharose, CNBr-activated Sepharose 4B, Sephadax G-10, DEAE Sephacel and Protein A conjugated Sepharose CL4B were from Pharmacia. NP-40 was from Particle Data Ltd. Polyethylene glycol, X-ray film and the Kodavue staining kit were from Kodak. Acrylamide and the silver staining kits were from BioRad. Prestained molecular weight markers were from Bethesda Research Laboratories, Bethesda, Maryland (BRL). $^{14}C$ methylated molecular weight markers, $^{3}H$-glucosamine, $^{35}S$-Methionine and $^{125}I$ were from Amersham. FITC-conjugated goat anti mouse immunoglobulin was from Tago. The Paragon serum protein electrophoresis apparatus was from Beckman. Ampholines were from LKB.

### Immunohistological Technique

For immunohistological studies on frozen sections, the unlabelled antibody technique of Sternberger in Immunochemistry, John Wiley & Sons, New York, 1979, pp:104-169, as modified by Garrigues et al. in Int. J. Cancer (1982) 29:511-515, was used. The target tissues for these tests were obtained at surgery and frozen within 4 hr of removal in isopentane which had been precooled in liquid nitrogen. Tissues were then stored in liquid nitrogen or at -70 °C until use. Rabbit anti-mouse IgG (Sternberger-Meyer Immunochemicals, Inc., Jarettsville, MD) was used at a dilution of 1/50. Mouse peroxidase-antiperoxidase complex (PAP, Sternberger-Meyer Immunochemicals, Inc.) containing 2 mg/ml of specifically purified PAP was used at a dilution of 1/80. Frozen sections were prepared, dried, treated with acetone and dried (Garrigues et al., 1982). Sections to be used for histologic evaluation were stained with hematoxylin. To decrease nonspecific background, sections were preincubated with normal human serum diluted 1/5 (Garrigues et al., 1982). Mouse antibodies, goat anti-mouse IgG, and mouse PAP were diluted in a solution of 10% normal human serum and 3% rabbit serum.

The staining procedure consisted of treating serial sections with either specific or control antibody for 2.5 hr, incubating for 30 min with rabbit anti-mouse IgG diluted 1/50, and exposing for 30 min to mouse PAP complex diluted 1/80. After each treatment with antibody, the slides were washed twice in phosphate buffered saline (PBS). The immunohistochemical reaction was developed with freshly prepared 0.05% 3,3'-diaminobenzidine tetrahydrochloride (Sigma, St. Louis, MO) and 0.01% hydrogen peroxide in 0.05 M Tris buffer, pH 7.6 for 8 min. Further exposure to a 1% $OsO_4$ solution in distilled water for 20 min intensified the stain. The sections were rinsed with water, dehydrated in alcohol, cleared in xylene, and mounted on slides.

The slides were each read under code and coded samples were checked by an independent investigator. Typical slides were photographed by using differential interference contrast optics (Zeiss-Momarski). The degree of antibody staining was evaluated as 0 (no reactivity), + (few positive cells), + + (at least one third of the cells positive), + + + (most cells positive), + + + + (close to all cells strongly positive). Since differences between + and 0 staining were less clear cut than between + + and + staining, it was decided to count as "positive" a staining graded as ++ or greater. Both neoplastic and stroma cells were observed in tumor samples; the staining recorded referred to that of the tumor cells, since the stroma cells were not stained at all, or were stained more weakly than the tumor cells.

Determination of Antigen Location

The subcellular localization of antigens was determined by measuring antibody binding to cells before or after permeabilization with non-ionic detergent. Antibodies binding to the cell surface of intact cultured cells were identified by either direct binding assays with $^{125}$I-labelled antibody (Brown et al., Proc. Natl. Acad. Sci. U.S.A. (1981a) 78:539-543) or by indirect fluorescence using the (FACS) II cell sorter. Antibodies binding to intracellular locations were determined by direct binding of $^{125}$I-labelled antibody to cells following fixation with paraformaldehyde and subsequent permeabilization with the non-ionic detergent NP-40.

Binding Assays

a) For binding assays performed by using radiolabelled antibodies (Brown et al., supra), cultured cells ($10^6$) were incubated at 4°C for 30 min with $10^6$ cpm of $^{125}$I-labelled antibody in 100 µl of heat-activated (30 min at 56°C) fetal calf serum in culture medium. After the addition of 5 ml of PBS, the cells were pelleted by centrifugation for 10 min at 250 X g. The supernatant was aspirated, and the pellet was counted for $^{125}$I. To measure nonspecific binding, parallel incubations were performed with 10 µg of unlabelled antibody as a competitor (Brown, et al., supra). In some instances binding assays were carried out in an analogous fashion on cells monolayers attached to plastic culture dishes.

b) For binding assays performed on the FACS II cell sorter, cells were removed from their substrata using PBS containing 5 mM ethylenediamine tetraacetic acid (EDTA). Samples containing 1 x $10^5$ cells were incubated first with monoclonal antibody at a concentration of 2 µg/ml followed by fluorescein-conjugated goat anti-mouse antibody at a 1:200 dilution. Cells were then washed and resuspended in culture medium. Immediately prior to FACS analysis, propidium iodide was added to a final concentration of 1 µg/ml to stain non-viable cells. During FACS analysis, cells emitting red fluorescence were electronically gated out so that only viable cells were examined. The mean intensity of fluorescein fluorescence was then determined for each antibody. Negative controls consisted of samples in which monoclonal antibody was omitted; positive controls consisted of monoclonal antibodies to HLA type 1 histocompatibility antigens. Staining was regarded as positive if the mean channel fluorescein was at least 3 times background.

Protein Antigen Determination

In order to identify protein antigens, lung carcinoma or human embryonic lung cells were surface radioiodinated or metabolically labelled with $^{35}$S-methionine. Antigens were isolated from cell lysates by incubation with monoclonal antibody, addition of goat anti-mouse IgG, and adsorption to S. aureus. Immune precipitates were washed and analyzed by sodium dodecyl sulfate-polyacrylamide gel electrophoresis (SDS-PAGE) (10-20% acrylamide) as described (Brown et al., supra).

$^{35}$S-Methionine Labeling

Hybridoma cells producing a particular antibody were seeded into a microtiter well in methionine free Dulbecco's Minimum Essential Medium (DMEN) containing 25 mM Hepes buffer, 4 mM L-glutamine, 4.5 gm/l glucose, 10 mM non-essential amino acids, 100 units/ml penicillin, 100 µg/ml streptomycin and 15% heat inactivated newborn calf serum (NCS). The cells were contacted for 6 hrs with 0.1 mCi $^{35}$S methionine at 37°C, the supernatant removed and centrifuged to remove cells.

Isotype Determination

a) Ouchterlony immunodiffusion

An aliquot of supernatant of particular hydridoma cells was placed into the center well of a 2% agar plate. Monospecific rabbit anti-mouse Ig isotypes antibodies (Meloy) were placed in the outer wells and the plate was incubated for 2 h at room temperature and overnight at 4°C.

b) Flexible polyvinylchloride 96 well plates (Costar) were coated with 0.1 mg/ml goat anti-mouse Ig antibodies for 2 h at 37°C and countercoated with a 3% BSA solution for 2 h at 37°C. The hydridoma supernatant was then incubated at 37°C for 2 h. After washing with PBS bovine serum albumin (BSA) plates were incubated at 37°C for 2 h with monospecific rabbit anti-mouse Ig isotype antibodies coupled to peroxidase (Zymed). After washing, plates were incubated with 1 mg/ml orthophenylenediamine and 0.03% $H_2O_2$ in 0.1 M citrate buffer pH 4.5. Optical density at 630 nm was determined on a Dynatec ELISA plate reader.

Staphylococcal Protein A Binding Assay

Microtiter wells were incubated with 5% NCS in PBS plus 0.02% $NaN_3$ and the supernatant was aspirated. Twenty-five µl of a suspension of epidermal cells ($2x10^7$ cells/ml) were added to each well and incubated with 25 µl of a particular antibody for 1 hr at room temperature. The plates were centrifuged at 1200 rpm for 7 min, washed twice with 50% NCS/PBS/$NaN_3$ and 25 µl $^{125}$I-staphylococcal protein A (about 50,000 cpm/25 l) were added. The plates were incubated for 1 hr at 25°C, washed twice with 5% NCS/PBS/$NaN_3$ and dried. The bottom of the wells were cut off and counted in a gamma counter.

Cell Culture

Calu-1 lung carcinoma cells were obtained from the American Type Culture Collection. Cells were maintained in monolayer cuture in Growth Medium (Dulbecco's Modified Eagle Medium (DMEM) containing 50 units of penicillin per ml, 50 µg of streptomycin per ml, and 10% (v:v) fetal calf serum (FCS)). Cells were collected by treatment with a solution of 0.05% trypsin and 0.02% EDTA and counted with a hemacytometer.

Indirect Immunofluorscence

Cells were collected by trypsinization and plated at a density of $5\text{-}20x10^3$ per well (6mm in diameter) on teflon coated multiwell slides (Meloy Labs). Cells were maintained at 37° for approximately 18 hrs prior to initiation of the experiment. The medium was aspirated and adherent cells were washed once with phosphate buffered saline (PBS, NaCl, 0.14M; KCl, 3mM; $Na_2HPO_4x7H_2O$, 8mM; and $KH_2PO_4$, 15mM) containing 0.5 mM $CaCl_2$ and 0.5 mM $MgCl_2$. Cells were then fixed in situ by the addition of 25 microliters of a solution of paraformaldehyde (0.5% in PBS) for 20-30 minutes at 23°. The wells were washed and intracellular antigens exposed by the addition of PBS containing 0.2% (v:v) NP40 for 5 min at 23°. Excess aldehyde groups were then blocked by addition of Binding Buffer (DMEM containing 50mM BES (N, N-bis [2-hydroxyethyl]-2-aminoethane sulfonic acid) at pH 6.8, 0.1% (w:v) BSA and 15% FCS) for a minimum of 15 minutes at 23°. Antibodies were diluted to a concentration of 10 µg per ml in Binding Buffer and applied in a total volume of 20-25 microliters per well. The slides were incubated at 4° for 1 hr and washed with binding buffer. A fluorescein isothiocyanate conjugatea (FITC) secondary antibody (goat anti-mouse IgG and IgM) was then added for 1 hr at 4°. The dilution of FITC conjugate used was determined experimentally as that yielding the optional signal to background ratio. Following this incubation, the slides were washed with Binding Buffer and $H_2O$ and air dried. A small volume of an anti-fade solution (Genetic Systens Corporation) and a coverslip were added and slides were examined using a Leitz Orthoplan fluorescence microscope. Slides were examined under a 40X oil immersion objection and photographed with Kodak Tri X Pan film.

Metabolic Labeling

A. $^{35}$S Methionine

Cells were collected by trypsinization. Viability, as determined by trypan blue enclusion, was usually greater than 95%. $4x10^6$ cells were collected by centrifugation, and suspended in a volume of 1 ml of Growth Medium, minus the amino acid methionine. $^{35}$S Methionine (800 Ci/mmole) was added to a final concentration of 0.5 mCi/ml and cells were incubated with rotation at 37° for period of 1-6 hrs. Labeled cells were then collected by centrifugation and washed prior to further manipulation. In some cases, the radiolabeled cell suspension was diluted ten-fold with Growth Medium containing unlabeled methionine and 10% FCS, added to a 100 mm tissue culture dish and incubated at 37° for an additional 18 hr. The adherent cells were then washed with PBS prior to use.

B. $^3$H glucosamine

A similar labeling protocol was used when labeling cells with $^3$H glucosamine. Internal glucose pools were first depleted by incubation in glucose-free Growth Medium containing 10% dialyzed FCS for a period of 4-24 hrs. Only cell populations which showed >90% viability following starvation were used for subsequent experiments. Cells were collected as described above and resuspended in a volume of 1 ml of glucose-free Growth Medium, containing 10% dialyzed FCS, 0.01M HEPES (N-2-Hydroxyethylpiperazine-N-2-ethanesulfonic acid) at pH 7.4, and 0.5 M Ci $^3$H glucosamine [40 Ci/mmole]. Following labeling at 37° with rotation for a period of 3 hrs, cells were collected by centrifugation and washed with PBS.

Iodinations

Proteins were radiolabeled with $^{125}$I using the chloramine T procedure as described by Greenwood, et al. (1963) Biochem. J. 89:114-123. Protein samples (50 $\mu$g) were diluted to 0.5 ml in PBS containing 1-2 m Ci $^{125}$I (carrier-free). Ten microliters of a freshly made solution of chloramine T (containing 10 $\mu$g) was added and the reaction was continued for a period of 5 min at 23°. The reaction was terminated by the addition of solutions containing 20 ug of $Na_2S_2O_5$, and 3 $\mu$g KI. Unreacted $^{125}$I was removed by chromatography on a column of Sephadex G-10® equilbrated with PBS containing 1mg/ml of bovine serum albumin. The specific activity of labeled monoclonal antibodies used in this study was approximately 3x10$^9$ cpm per nanomole. Samples containing L3 antigen were desalted on a spin column of Sephadex G-10 prior to radiolabeling.

Immunoprecipitation Analysis

1. Preparation of Extract

Metabolically labeled cells were collected by centrifugation and solubilized in RIPA buffer containing NaCl, 0.15M; $Na_2HPO_4$, 0.05 M; sodium deoxycholate, 1% (w:v); Triton X-100, 1% (w:v), and SDS, 0.1% (w:v)) at a ratio of 2-4x10$^6$ cells per milliliter. Following incubation for 10 min at 4°, the mixture was clarified by centrifugation (147,000 x g for 30 min, Ti 70.1 rotor). The supernatant was removed and incorporation of radioactivity was determined by liquid scintillation counting.

2. Immunoprecipitation

Aliquots of cell lysate containing 1-2x10$^7$ cpm (acid precipitable) for $^{35}$S-methionine and $^{125}$I or 4x10$^6$ cpm (acid precipitable) for $^3$H glucosamine were incubated with 1 microgram of antibody for 0.5 - 1 hr at 4°. When labeled culture medium was analyzed, an aliquot was used which was equivalent to the percentage of the total cell extract analyzed. A solution containing affinity purified goat antimouse immunoglobulin (1-2 $\mu$g; prepared by immunizing a goat with mouse immunoglobulin) was added and incubation at 4°C was continued for an additional 10-60 min. Fifty microliters of a 10% solution of formalin fixed Staphlococcus aureus (Pansorbin) was added, and following a 5 min incubation at 4°C, immunoprecipitates were collected by centrifugation. (Before use, Pansorbin was washed once in a solution of 0.5% (v:v) NP40, once in a solution of 0.05% (v:v) NP40 and resuspended in the latter buffer containing 1 mg/ml ovalbumin.) Precipitates were washed 3-4 times in TNEN buffer (Tris hydroxymethylaminomethane, pH 8.0, 0.02M; NaCl, 0.1M; EDTA, 0.01M; and NP-40, 0.5% (w:v)) and stored frozen at -20°C until use.

Electrophoresis

1. SDS-Polyacrylamide Gel Electrophoresis (SDS PAGE):

SDS PAGE was run according to Laemmli, supra. The resolving gel (0.75mm) used most frequently was 10-20% linear acrylamide gradient and the stacking gel contained 5% acrylamide. Samples were boiled in sample buffer containing 5% 2-mercaptoethanol, and the immunoabsorbent was removed by centrifugation prior to electrophoresis. Molecular weight standards were either a $^{14}$C-methylated protein mixture (Amersham) or prestained standards (BRL). The molecular weights of the standard proteins used were myosin (200,000), phosphorylase b (92,400), bovine serum albumin (BSA) (69,000), ovalbumin (46,000), chymotrypsinogen (26,000), betalactoglobulin (18,400), lysozyme (14,300) and cytochrome c (12,300). After electrophoresis, gels were dried in vacuo and positions of radiolabeled proteins were determined by autoradiography on Kodak X-ray film.

2. Two Dimensional isoelectric focusing (IEF)-SDS PAGE:

Immunoprepcipates containing $^{125}$I-labeled L3 antigen were prepared as described, and resuspended in 50 microliters of a solution containing 5% (v:v) 2-mercaptoethanol and 1% (v:v) NP40. Samples were heated at 95°C for 5 min and the immunoabsorbent was removed by centrifugation. Samples were mixed with ampholines (pH range 3.5-10; final concentration 4%) and solid urea was added to saturation. Isoeletric focusing and SDS Page were run according to the procedure of O'Farrell (1975) J. Biol. Chem. 250:4007-4021. Standard protein markers (BioRad) were used to monitor separation in the IEF dimension. The resulting pH gradient was determined from a blank gel run in parallel. The gel was sliced into 0.5cm slices, the slices soaked in 1 ml distilled $H_2O$ for 1 hr and the pH of the resulting solution determined.

3. Paragon electrophoresis:

Serum protein electrophoresis was conducted using preformed agarose gels on a Paragon apparatus (Beckman) according to the manufacturers instructions.

Immunoblotting:

Proteins to be analyzed were separated by either SDS PAGE or Paragon. Samples separated by SDS PAGE were electrophoretically transfered to nitrocellulose as described by Burnette (1981) Anal. Biochem. 112:195-203 at 5 V/cm for a period of approximately 18 hr at 4°. Samples separated by Paragon were transfered to nitrocellulose by overlaying the electrophoretogram with a sheet of nitrocellulose paper. Nitrocellulose was hydrated in the transfer buffer of Burnette, supra, without SDS before use. A stack of paper towels approximately 1 inch thick was placed over the nitrocellulose and weighted down with a glass plate and reagent bottle. Transfer was complete within a 1 hr period, as judged by staining of the gel following transfer. Nitrocellulose blots were blocked by a 1 hr incubation in Blotto, a milk based coctail (Johnson et al., Gene Anal. Technol. (1984) 1:3-8) (PBS containing 1% (w:v) Carnation non-fat dry milk and 0.2% (v:v) NP-40). L3 antigen was revealed by incubation of the blots with 1-4x$10^6$ cpm/ml $^{125}$I-L3 antibody in Blotto for 1 hr at 23°. Blots were washed extensively with Blotto, and dried before autoradiographic exposure.

Binding of $^{125}$I-L3 Antibody

Calu-1 cells (4-10x$10^4$) were plated in 48 well cell culture dishes 18 hr prior to the initiation of the experiment. Cells were washed with PBS and fixed with a solution of 0.5% paraformaldehyde in PBS for a period of 20 min at 23°C. Monolayers were washed with PBS and permeabilized by treatment with PBS containing 0.2% NP-40 for 5 min at 23°. Various concentrations of $^{125}$I labeled antibody were added in a final volume of 0.1 ml for a period of 60 min at 23°. Monolayers were then washed extensively with binding buffer, and solubilized by the addition of 0.5 M NaOH. Cell bound radioactivity was determined on a gamma counter. Samples were assayed in duplicate; duplicate samples generally varied by less than 20%. To measure non-specific antibody binding, indentical wells were incubated with 50-100 fold excess of unlabeled L3 antibody, which usually reduced binding of $^{125}$I-labeled antibody by more than 90%. When binding data was analyzed according to Scatchard (1949) Ann. N.Y. Acad. Sci. 51:660-672, an affinity constant ($K_2$) of approximately 1x$10^8$ $M^{-1}$ was determined for $^{125}$I L3 antibody. Binding of antibody was reduced by more than 10 fold if cell monolayers were not treated with NP40 prior to addition of $^{125}$I-L3 antibody.

Competitive binding assay

L3 antigenic activity was measured by determining the ability of the antigen to inhibit binding of $^{125}$I-labeled L3 antibody to formalin fixed monolayers of Calu-1 cells. Cell monlayers were prepared as described above. $^{125}$I-L3 antibody was added to cells at a concentration of 0.4 ug/ml in the presence or absence of solutions containing L3 antigenic activity. The final assay volume was 0.1 ml. Under these conditions, the binding of $^{125}$I-L3 antibody was more than 95% specific, as judged by the ability of unlabeled L3 antibody to compete for binding. Various dilutions of the test solutions were added and binding of $^{125}$I-labeled antibody was measured as described above. Typical inhibition curves for various stages of the purification of L3 antigen found in culture medium and in normal human serum are shown in Figure 1, Supplemental Material. One unit of L3 antigenic activity is defined as the amount necessary to inhibit the binding by 50% of $^{125}$I-L3 antibody added at 0.4 ug/ml.

Glycosidase treatments

Immunoprecipitates of $^{35}$S-methionine or $^{125}$I-labeled L3 antigen were prepared as described. The pelleted immunoabsorbant was resuspended in a volume of twenty five microliters of digestion buffer, a volume of five microliters containing 5 milliunits of the appropriate enzyme was added and the samples were incubated at 37° for 18 hr. Concentrated SDS PAGE sample buffer was added and samples were subjected to electrophoresis. For neuraminidase treatment, the digestion buffer contained: NaCl 0.15 M; sodium acetate at pH 5.3, 0.05 M; $CaCl_2$, 0.1% (w:v); and phenyl methyl sulfonyl fluoride, 0.1% (w:v). For endoglycosidase H (Endo H) treatment the digestion buffer contained: Tris-HCl at pH 6.5; 0.025 M; SDS, 0.2% (w:v); and phenylmethylsulfonyl fluoride, 0.1% (w:v).

Protein determination

Protein concentrations were determined by the method of Bradford (1976) Anal. Biochem. 72:248-254 using bovine serum albumin as a standard.

Preparation of L3 Sepharose

Lyophilized CNBr-activated-Sepharose 4B® was reconstituted in 1mM HCl at 4° for a period of 30-60 min. The resin was washed once in the same solution, collected by centrifugation, mixed with L3 antibody at 3.5 mg/ml in a solution of 1 M NaCl and 0.2 M sodium bicarbonate at pH 8.3. The mixture was incubated at 4° with rotation for a period of 16 hrs. The resin was collected by centrifugation and excess active groups were blocked by the addition of 1-4 volumes of a solution of 0.25 M glycine at pH 8.3. The resin was then collected by centrifugation and washed several times in PBS before use. Efficiency of coupling was assessed by measuring absorbance at 280 nm of the antibody solution before and after coupling, and was determined to be approximately 10 mg L3 antibody per ml of packed resin.

Gel staining

After SDS PAGE, positions of protein bands were determined by staining with Coomassie blue, or on commercial silver (BioRad) or nickel (Kodak) based staining kits.

Amino-Terminal Sequencing by Edman Degradation

L3 antigen from culture medium was reduced with dithiothreitol (20mM) in a volume of 0.1 ml of a solution containing 0.1 M Tris-HCl, pH 8.5 and 0.1% SDS (w:v) for 2 hrs at 50°. The antigen was then S-carboxamidomethylated by treatment with iodoacetamide (45 mM) for 30 min. at 20° and subjected to preparative SDS PAGE on a 10% acrylamide gel (1.5 mm thick). Following electrophoresis, the gel was stained with Coomassie Brilliant Blue (0.5% by weight in 10% acetic acid and 30% isopropanol) and destained in a solution of acetic acid (5% v:v) and methanol (17% v:v). The stained L3 antigen band was excised with a razor blade and immediately subjected to electroelution and electrodialysis with a ECU-040 Electroelutor/Concentrator (C.B.S. Scientific Co., San Diego, Ca). Serum-derived L3 antigen was processed identically, except that the sample was not carboxamidomethylated. Overall recovery of the procedure was approximately 80%, based on estimating the amount of protein by analytical SDS-PAGE with silver staining. Automated Edman degradation was performed with approximately 100 pmol of S-carboxamidomethylated conditioned medium-derived L3 antigen (based on the yield of identified Met-6) and 38 pmol of serum-derived unmodified L3 antigen (based on the yield of identified Val-1) in a gas-phase sequencer, (Model 470A, Applied Biosystems, Inc. Foster City, Ca). The phenylthiohydantoin amino acids were analyzed by reverse-phase HPLC (detection Unit, 2 pmole) with an IBM Instruments' Cyano column using a sodium acetate buffer/Tetrahydrofuran/acetonitrile gradient for elution (Applied Biosystems, Protein Sequence Users Bulletin No. 2, 1984).

## EXAMPLE 1

### Preparation of Monoclonal Antibodies

Monoclonal antibodies were produced by immunizing 3-month-old BALB/c mice with human tissues of one of four different sources: (1) pleural effusions from patients with metastic non-small cell lung carcinoma, (2) cultured cells from a non-small cell lung carcinoma, and (3) lung tissue from 3-4 months-old human embryos. The immunizations were performed by injecting the mice intraperitoneally 3-4 times with approximately $10^7$ cells. Three days after the last immunization, the spleens were removed, suspended in culture medium and fused with NSI mouse myeloma cells (Köhler and Milstein, supra). The mixtures were seeded to form low density cultures originating from single fused cells (clones); the techniques used for the hybridization have been previously described by Yeh, et al., Int. J. Cancer (1979) 29:269-275.

Supernatants from hybrid cells were screened by using both an ELISA assay and an autoradiographic indirect $^{125}$I-labelled protein A assay (Brown et al., J. Immunol. Meth. (1979) 31:201-209 against extracts from the tumors used for immunization which contained, i.a., cell membranes. These extracts were prepared using a procedure modified from Colcher et al., Cancer Res., (1981) 42:1451-1459; Yeh et al., supra. For this, tissues were washed with PBS and suspended, which for intact tumors was done by pressing through a stainless steel screen. After this 1 mM $NaHCO_3$ containing 1 mM phenylmethylsulfonylfluoride (Calbiochem-Behring Corp., San Diego, CA) was added, and the material was then homogenized on ice, with 50 strokes of the B pestle of a Dounce homogenizer. After centrifugation for 15 min at 27,000 x g, the supernatant was removed, and the pellet was resuspended in PBS, sonicated for 1 min, and stored at -70 °C.

Hybridomas which produced antibodies binding to the cell membrane extracts were cloned, expanded in vitro, and further tested for antibody specificity. This testing was carried out by using the Immunohistological Technique described above, in which the ability of the antibodies to bind to frozen sections of lung carcinomas, other tumors and normal human tissues were tested. Those hybridomas which produced antibody of apparent specificity for human lung cancer were recloned, expanded and injected into pristane-primed 3-month old BALB/c mice, where they grew as ascites tumors.

Antibodies secreted into the ascites were purified on protein A Sepharose (Ey et al., Immunochemistry - (1978) 15:429) or by gel filtration in Sephacryl S-300®. Purified antibodies were used for further characterization which included additional specificity tests by immunohistology, binding assays on intact cells to determine which antibodies bound to the cell surface, and the radioimmunoprecipitation tests as described above.

Monoclonal antibodies L3, and L5, were produced from the corresponding hybridomas as described above. These antibodies exhibited the properties indicated above in this specification.

The cell lines, designated L3, and L5, were deposited at the A.T.C.C. (American Type Culture Collection, 12301 Parklawn Or., Rockville, Maryland 20852) on October 4, 1984, and received accession numbers HB8625 and HB8627, respectively. Cell line L3 was redeposited on October 25, 1984.

## EXAMPLE 2

### Purification of L3 Antigen from Serum Free Culture Medium

#### 1. Preparation of serum-free conditioned medium

Calu-1 cells were grown to confluence in roller bottles (850 $cm^2$) in the presence of Growth Medium. Monolayers were washed 3 times with 50 ml of serum-free Growth Medium, the second wash being left on the cells for 30 min at 37 °C. Cells were then incubated in the presence of 150 ml serum-free Growth Medium. After 3 days, the medium was collected, centrifuged to remove floating cells, and stored frozen at -70 °C until use.

#### 2. Preparation of concentrate

Serum-free medium (790 mls) was concentrated to a volume of 17 ml by ultrafiltration (Amicon, PM10 membrane). The concentrate was removed and the membrane was washed once with PBS (11ml). The wash and concentrate were combined and the mixture was centrifuged at 147,000 x g for 30 min in a Ti70 rotor (Beckman). The supernatant was found to contain most of the L3 antigenic activity and is hereafter referred to as the concentrate.

3. Immunoaffinity Chromatography

The concentrate was passed over a column (1.5ml) of Sepharose Cl4B® to remove substances having affinity for Sepharose. The column was then washed with 3 mls of PBS. The flow-through was combined with the wash and added to a packed volume of 1 ml of L3 antibody conjugated Sepharose 4B (10mg L3/ml resin). The mixture was held at 4°C with rotation for a period of 18 hr, and then poured into a column made from a disposition hypodermic syringe. The flow through was collected and the resin washed with PBS (10 ml), and solutions of 5M NaCl (5 ml) and 0.02 M ammonium acetate (10ml). L3 antigen was eluted from the column by the addition of a freshly made solution of triethylamine (75mM). Since L3 antigenic activity was labile at elevated pH, the pH of the effluent was adjusted by collecting fractions (0.5 ml) in tubes containing 0.1 ml of 2M ammonium acetate. Fractions containing L3 activity were identified using the competitive binding assay, pooled, and stored frozen at -20°C. The results are summarized in Table I.

EXAMPLE 3

Purification of L3 Antigen from Serum

1. Collection of Serum

Blood was drawn from tumor patients or normal individuals and allowed to clot from 10 to 90 minutes at room temperature. Samples were then stored at 4-6°C from 30 min to 5 hr before centrifugation for 10 min. at 1600 rpm in a Sorvall Clinical centrifuge. Serum was separated from the clot, aliquoted, and stored frozen at -70°C until use. Levels of L3 antigenic acitivity did not differ significantly in serum or plasma from the same individual. Thawed aliquots were generally not refrozen since antigenic activity was found sensitive to repeated freeze-thawing. For purification of L3 antigenic activity an aliquot of 10ml freshly frozen serum was thawed and clarified before use by centrifugation at 147,000 x g for 60 min (Ti 70.1 rotor).

2. DEAE Sephacel Chromatography

A 10 ml column of DEAE Sephacel® was poured and equilibrated with PE buffer (0.02 M Sodium phosphate, pH 7.12 and 0.005 M EDTA). Clarified serum was diluted to a volume of 130 ml with PE buffer before application to the ion exchange column. A flow rite of 0.5 ml/min was maintained during sample application and elution. The column flow through contained 54% of the initial serum protein (as judged by absorbance at 280 nm) and no detectable L3 antigenic activity. The column was then washed with 10 column volumes of PE buffer. Elution of L3 antigenic activity was achieved with a 50 ml gradient of 0 to 0.5 M NaCl in PE buffer. Fractions of 1 ml were collected during elution. Following application of the gradient, the column was washed with 0.5 M NaCl in PE and finally with 1.5 M NaCl in the same buffer. Fractions containing L3 antigenic activity were identified using the competitive inhibition assay and pooled. L3 antigenic activity trailed slightly behind the bulk of eluted serum proteins.

In some experiments L3 antigenic activity was partially purified prior to radiolabeling and immunoprecipitation analysis by a slightly different procedure. Serum proteins were first precipitated with 30% ammonium sulfate. The precipitate was dissolved in $H_2O$ and desalted (on Sephadex G-25®) before application to a column of DEAE Sephacel®. Elution of L3 activity was achieved by stepwise addition of solutions of NaCl. A fraction eluting between 0.25 and 0.5M NaCl was enriched for L3 antigenic activity and was used for subsequent radiolabeling of L3. Antigen prepared in this fashion was indistinguishable by protease fingerprinting from radiolabeled antigen precipitated from preparations more than 3,000 fold purified.

3. Immunoaffinity chromotography

Pooled fractions from a DEAE Sephacel column (14 ml) were applied to a 8.4 ml column of Sephacel CL 4B® and the column was washed with 17 ml of PBS. The wash was then combined with the flow through and the mixture equilibrated 18 hrs with 1.5 ml packed volumn of antibody L3 conjugated Sepharose (10 mg L3/ml resin) by rotation at 4°C. The mixture was poured into a column and the resin washed sequentially with PBS (42 mls), PBS containing 0.2% (v:v) NP40 (28 ml) PBS (14ml), 0.02 M ammonium acetate (28 ml), 5M NaCl (28 ml) and finally, 0.02 M ammonium acetate (5 ml). L3 antigen was eluted by addition of 8.4 ml of 0.075 M trielthylamine as described above. The results are summarized in Table II and III.

EXAMPLE 4

Competitive Binding of L3 Antibody and Antibody 465.12S

Either unlabeled antibody 465.12S or unlabeled L3 antibody were mixed with $^{125}$I-L3 antibody (final concentration, 0.35 ug/ml) and added to formalin-fixed H125 lung carcinoma cells (50,000/well). Unlabeled antibodies were used as hybridoma supernatants; each was diluted to one-half the original volume in the final assay. The solution of antibody 465.12S used contained sufficient antibody to precipitate, from $^{35}$S-méthionine labeled cell extracts, a quantity of antigen equivalent to that precipitated by one microgram of purified L3 antibody. The results shown in Table IV indicate that 465.12S did not compete for binding of $^{125}$I-L3 antibody. Thus, the two antibodies recognize distinct epitopes and are therefore different antibodies and not functional equivalents.

EXAMPLE 5

Cleavage Fragments of L3 Antigen

CALU-1 human lung carcinoma cells were surface labelled using lactoperoxidase catalyzed iodination as described by Brown et al. (J. Immunol. 127: 539-546, 1981). The L5 antigen (that is the antigen defined by antibody L5) was precipitated from cell extracts and analyzed by SDS-PAGE run under reducing conditions. When analyzed accordingly, the L5 antigen migrated as a single polypeptide chain of $M_r$ 135,000. The region of the gel containing the L5 antigen was excised, treated with the indicated amount of Staphylococcus aureus V8 protease, and analyzed again by SDS-PAGE, as described by Cleveland et al., supra. The resulting "fingerprints" of cell surface iodinated V8 cleavage fragments are diagnostic for the L5 antigen, i.e., fragments of $M_r$ 24,000 and 16,000 are characteristic of the L5 antigen and distinguish the L5 antigen from other antigens of similar molecular weight.

Table I

Purification of L3 Antigenic Activity from Culture Medium of Calu-1 Cells

| Sample | Volume (ml) | Protein[1] (mg) | Activity[2] (U) | Yield (%) | Specific Activity (U/mg) | Purification factor |
|---|---|---|---|---|---|---|
| Medium | 910 | 35.5 | 18,600 | 100 | 522 | 1 |
| Concentrate | 27.8 | 11.9 | 12,900 | 70 | 1090 | 2.08 |
| L3 affinity column | 4.2 | 0.139 | 6,470 | 35 | 46500 | 89.1 |

Table II

Purification of L3 Antigenic Activity from Normal Human Serum

| Sample | Volume (ml) | Protein[1] (mg) | Activity[2] (U) | Yield (%) | Specific Activity (U/mg) | Purification factor |
|---|---|---|---|---|---|---|
| Serum | 10 | 880 | 11,800 | 100 | 13.3 | 1 |
| DEAE pool | 14 | 118 | 9,040 | 79 | 79 | 5.9 |
| L3 affinity column | 10 | 0.1 | 4,550 | 39 | 45,500 | 3420 |

1) Determined by the method of Bradford (1976) Anal. Biochem. 72:248-254.

2) Amount required to inhibit binding of $^{125}I$-labeled L3 antibody by 50% in the competitive binding assay described in the Examples.

Table III

| Characteristics of the L3 Antigen in Normal Human Serum | |
|---|---|
| CONCENTRATION: | 5.8 ± 2.6 ug/ml[1] |
| MOLECULAR WEIGHT: | 94,000 Form I<br>76,000 Form II<br>26,000 Form III |
| MOBILITY<br>pI:<br>N-TERMINAL AMINO ACID | alpha-2<br>4.2-5.0 (Forms I and II)<br>Val (Form I) |

[1]Determined as the mean value (± standard deviation) from seven nomal healthy individuals. Sera were tested at a final concentration of 5.7% by volume in the competitive binding assay, using immunoaffinity purified L3 antigen from culture medium as a standard. Values were corrected for the purity of the standard preparation which was estimated (by densitometric scanning of a silver stained SDS PAGE gel) to be 66%.

TABLE IV

| Unlabeled Antibody | $^{125}$I-L3 Antibody Bound (cpm) |
|---|---|
| None | 9524 (100) |
| 465.12S | 10028 (105) |
| L3 | 1194 ( 12) |

The invention has been described in detail with particular reference to the above embodiments. It will be understood, however, that variations and modifications can be effected within the spirit and scope of the invention.

## Claims

1. An antigenic composition purified from the culture medium of human non-small cell lung carcinoma cells comprising a non-denatured glycosylated Form I L3 protein, said protein having the following N-terminal amino acid sequence:

```
1         5              10             15             20            25            30
V-N-D-G-D-M-R-L-A-D-G-G-A-T-N-Q-G-R-V-E-I-F-Y-R-G-Q-W-G-T-V,
```

a molecular weight of about 94,000 daltons as determined by SDS polyacrylamide gel electrophoresis under reducing conditions, and N-linked glycoside residues in which the terminal residues of the N-linked glycoside residues are sialic acid residues.

2. An antigenic composition purified from normal human serum comprising a non-denatured glycosylated Form I L3 protein, said protein having the following N-terminal amino acid sequence.

```
1         5              10             15             20            25            30
V-N-D-G-D-M-R-L-A-D-G-G-A-T-N-Q-G-R-V-E-I-F-Y-R-G-Q-W-G-T-V,
```

a molecular weight of about 94,000 daltons as determined by SDS polyacrylamide gel electrophoresis under reducing conditions, and N-linked glycoside residues in which the terminal residues of the N-linked glycoside residues are sialic acid residues.

3. The composition of claim 1 or 2, which further comprises an L3 Form II protein having a molecular weight of about 76,000 daltons as determined by SDS polyacrylamide gel electrophoresis under reducing conditions.

European Patent Office

# EUROPEAN SEARCH REPORT

Application Number

EP 93 10 6003

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | CANCER RESEARCH<br>vol. 42, February 1982, PHILADELPHIA<br>pages 583 - 589<br>NATALI, P.G. ET AL 'Tisuue distribution, molecular profile, and shedding of a cytoplasmic antigen identified by the monoclonal antibody 465.12S to human melanoma cells'<br>* the whole document *<br>--- | 1-3 | C07K15/06<br>A61K39/00<br>//C12P21/08<br>G01N33/577 |
| T | BIOCHEMISTRY.<br>vol. 25, no. 10, 1986, EASTON, PA US<br>pages 2978 - 2986<br>LINSLEY, D.H. ET AL 'Identification of a novel serum protein secreted by lung carcinoma cells'<br>*page 2981, results, "the L3 antigen is identical with antigen recognized by antibody 465.12S"*<br>* page 2985; table III *<br>----- | 1-3 | |

| TECHNICAL FIELDS SEARCHED (Int. Cl.4) |
|---|
| C07K<br>A61K |

The present search report has been drawn up for all claims

| Place of search | Date of completion of the search | Examiner |
|---|---|---|
| THE HAGUE | 01 JULY 1993 | FERNANDEZ Y BRA F. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)